# EUROPEAN PATENT APPLICATION

(11) **EP 1 839 641 A1**
(43) Date of publication of application: **03.10.2007**
(21) Application number: 06005864.1
(22) Date of filing: 22.03.2006
(51) Int. Cl.: A61K 8/00

(54) **Cosmetic composition comprising botanical extracts and a thickener**

(71) Applicant: THE PROCTER & GAMBLE COMPANY, Cincinnati, Ohio 45202 (US)
(72) Inventor: Gibbons, Clyde, Staines, Middlesex TW18 3DE (GB); Watson, Andrew David, Redhill, Surrey, RH1 6ND (GB); Morris, Sian, Berkshire RG12 0UZ (GB)
(74) Representative: Wilding, Richard Alan

(57) **Abstract**

A cosmetic composition is provided in the form of an oil-in-water emulsion comprising a continuous aqueous phase comprising a cross-linked co-polymer and a hydrophilic botanical extract. A process suitable for preparing that composition and the use of that composition are also provided.

## Description

### TECHNICAL FIELD OF THE INVENTION

Cosmetic compositions in the form of oil-in-water emulsions that comprise a hydrophilic botanical extract in combination with a cross-linked co-polymer are provided, said hydrophilic botanical extracts exhibiting higher stability and efficacy. A process for preparing said compositions and the use of said compositions is also provided.

### BACKGROUND OF THE INVENTION

Botanical extracts are commonly used in cosmetic compositions to prevent, regulate and/or to treat various conditions of the skin. Nevertheless, since each botanical extract generally comprises many widely differing components, it may be difficult to formulate them into a composition and, once formulated, those extracts can sometimes exhibit a lower efficacy than expected, for a number of reasons, such as method steps involved in preparing the composition and/or the denaturation of the extract components with time. Moreover, those extracts, once applied onto the skin, can cause irritation to the skin, said irritation being generally due to the extract's degradation products.

It is generally known that cosmetic compositions may comprise various types of ingredients, such as carriers, gelling agents, suspending agents, thickeners. Those ingredients are chosen in order to confer specific properties to the cosmetic compositions (solubility, viscosity, etc.). Thickeners are commonly use in cosmetic compositions to change the viscosity and/or the tackiness of cosmetic compositions. For example, FR 2 840 809 discloses a composition comprising a hydrophilic extract of Seabuckthorn and a acrylate/C10-30 acrylate crosspolymer.

Many other thickeners are known such as the new thickener system disclosed in US 6,099,829. That new thickener system is in the form of a cross-linked, water-swellable polymer comprising; i) from 35% to 100% by weight of ionic monomers; ii) from 0% to 65% by weight of non-ionic monomers; and iii) from 0.3 to 1mol%, based on (i) and (ii), of one or more at least bifunctional monomer, that polymer permitting simple formulation of cosmetic or dermatological preparations such as water-in-oil emulsions exhibiting good efficacy, easy wettability, self-inverting, able to be used without an additional neutralizing step, and giving a pH of 6-7. In particular, that polymer is used to increase the viscosity of compositions without impairing the application feel.

There is a constant need to develop compositions in which botanical extracts exhibit a high stability without disadvantageous side effects. More precisely, there is a need to prevent the denaturation and/or to improve the safety and the efficacy of hydrophilic botanical extracts during the process for preparing the composition, during the storage and once applied onto the skin.

Unexpectedly, the inventors have found that that cross-linked, water-swellable polymer, when used in cosmetic oil-in-water emulsions comprising a hydrophilic botanical extract, impacts on both stability and efficacy of these hydrophilic botanical extracts.

### SUMMARY OF THE INVENTION

According to a first aspect of the invention, a cosmetic composition is provided in the form of an oil-in-water emulsion, wherein said emulsion comprises a continuous aqueous phase comprising (1) a cross-linked co-polymer comprising; i) from 35% to 94.5% water-soluble anionic acrylic monomers by weight of the cross-linked co-polymer; ii) from 5% to 65% water-soluble non-ionic acrylate monomers by weight of the cross-linked copolymer; and iii) from 0.005% to 0.5% bifunctional monomeric crosslinking agent by weight of the cross linked co-polymer, and (2) a hydrophilic botanical extract. The composition of the present invention comprises hydrophilic botanical extracts exhibiting increased stability and/or efficacy.

The present inventors have now surprisingly found that hydrophilic botanical extracts in combination with said cross-linked co-polymer exhibit a higher stability and/or efficacy. Without wishing to be bound by theory, it is believed that said cross-linked co-polymer unexpectedly prevents the denaturation of hydrophilic botanical extracts both during the process for preparing the composition, during its storage and once applied to the skin.

### DEFINITIONS

As used herein, the word "hydrophilic" in relation to the material means that that material is above 10% soluble in water by weight at standard temperature and pressure (STP).

As used herein, the word "hydrophobic" as used in relation to a material means that that material is less than 0.1% soluble in water by weight at standard temperature and pressure (STP).

As used herein, the expression "oil-in-water emulsion" means that the composition has a continuous aqueous phase and a hydrophobic, water-insoluble ("oil") phase dispersed therein.

These and other features, aspects, and advantages of the present invention will become evident to those skilled in the art from a reading of the present disclosure.

### DETAILED DESCRIPTION OF THE INVENTION

The cosmetic composition is in the form of an oil-in-water emulsion comprising a continuous aqueous phase comprising (1) a cross-linked co-polymer comprising (i) from 35% to 94.5%, preferably from 50% to 70%, water-soluble anionic acrylic monomers, (ii) from 5% to 65% water-soluble non-ionic acrylate monomers by weight of the cross-linked co-polymer, and (iii) from 0.005% to 0.5% bifunctional monomeric cross-linking agent by weight of the cross-linked co-polymer, and (2) a hydrophilic botanical extract.

Said cross-linked co-polymer, comprising (i) from 35% to 94.5%, preferably from 50% to 70%, water-soluble anionic acrylic monomers, (ii) from 5% to 65% water-soluble non-ionic acrylate monomers by weight of the cross-linked co-polymer, and (iii) from 0.005% to 0.5% bifunctional monomeric cross-linking agent by weight of the cross-linked copolymer, has been disclosed previously in US 6,099,829.

Suitable water-soluble anionic acrylic-based monomers include acrylic acid, methacrylic acid or mixtures thereof. Suitable water-soluble non-ionic acrylate-based monomers include acrylamide, methacrylamide, N-vinyl pyrolidone, water-soluble hydroxy-substituted acrylic or methacrylic esters or mixtures thereof. Suitable bifunctional monomeric cross-linking agents include di-, tri- and tetraethylenically unsaturated materials such as methylene bis acrylamide, divinylpyrroline and allyl(meth)acrylate or mixtures thereof.

The cosmetic composition may comprise from 0.01% to 10%, preferably from 0.1% to 7%, more preferably from 1% to 3% of cross-linked co-polymer by weight of the composition.

Commercial examples of co-polymer compositions suitable for use herein include the copolymer compositions commercially available from BASF Corp. under the trade name Luvigel® EM.

The composition of the present invention further comprises a hydrophilic botanical extract. Preferably, the hydrophilic botanical extracts comprises hydrophilic extracts of aloe vera leaf (*aloe vera*), argan tree leaf and/or fruit (*argania spinosa*), baobab or Cream of Tartar tree leaf (*adansonia digitata*), bearberry leaf (*arctostaphylos*), brahmi (or water hyssop) whole plant (*Bacopa monnieri*), butcher's broom (*ruscus root*), centella asiatica (*Centella asiatica*), chamomile (*Chamomilla Recuita matricaria*), chestnut (*castanea sativa*), clary sage leaf (*salvia sclarea*), eyebright leaf (*euphrasia officinalis*), ginkgo biloba leaf (*ginkgo biloba*), grape fruit, leaf and/or seed (*vitis vinifera*), green tea leaf (*Camellia Sinensis*), honey (*mel*), horestail leaf (*equisetum arvense*), horse chestnut seed (*aesculus hippocastanum*), Indian cress whole plant (*tropaelolum ajus*), lemongrass plant leaf (*cymbopogon Schoenanthus*)*,* linden wood, leaf and/or bark (*Tilia Cordata*), liquorice root (*glycyrrhiza glabra*), marigold flower (*calendula officinalis*), mimosa leaf and/or bark (*mimosa tenuiflora*), mulberry leaf (*morus nigra*), neem leaf and/or bark (*azadirachta indica*), nettle leaf (*urtica dioica*), oat germ and/or bran (*Avena sativa*), oergano leaf (*Origanum officinalis*), olive leaf and/or fruit (*Olea Europaea*), panax ginseng root extract (*Panax Ginseng*), plantago leaf (*plantago lanceolata*), propolis (*propolis*), rose hip fruit (*Rosa canina*), rosemary leaf (*Rosmarinus*), sage leaf (*salvia officinalis*), st. Mary's thisle (*silybum marianum*), seabuckthorn (*Hippophae rhamnoides*), sesame seed (*Sesamum indicum*), sweet manadarin peel (*citrus nobilis*), wheat bran and/or germ (*triticum vulgare*)*,* willow bark (*Salix alba*)*,* witch hazel (*hamamelis Virginia*) or mixtures thereof, preferably an hydrophilic extract of rose hip fruit, ginko biloba leaf, panax ginseng root, seabuckthorn or mixtures thereof. For example, a suitable product containing a hydrophilic extract of Seabuckthorn is commercially available under the name FRUITAPONE® SEABUCKTHORN B from Symrise (INCI name: propylene glycol, aqua, citric acid, Hippophae Rhamnoides, trideceth-9, bisabolol), said product comprising 5% of hydrophilic extract of Seabuckthorn.

The cosmetic composition according to the invention may comprise from 0.0001% to 1%, preferably from 0.001% to 0.4%, and more preferably from 0.002% to 0.15% of a hydrophilic botanical component by weight of the composition.

Without wishing to be bound by theory, it is believed that the addition of said cross-linked co-polymer to the cosmetic composition may increase the stability and the efficacy of said hydrophilic botanical extracts during the formulation, the storage and the use/application of that composition. Particularly, it is believed that the enhancement of stability and/or efficacy of said extracts may be attributable to a reduction in denaturation of said extracts. As will be understood, it is advantageous to limit the denaturation of the hydrophilic botanical extract because the same product efficacy may be achieved with less extract. Preventing denaturation of the hydrophilic botanical extract may also reduce the discoloration of the product, the generation of odours and skin irritation caused by degradation by-products. Furthermore, it is believed that these unexpected effects may be obtained by the fact that both said polymer and said extracts are included in the same phase, i.e. the aqueous phase.

Cosmetic compositions according to the invention further comprise at least one humectant. Preferably, the humectant comprises polyhydric alcohols. More preferably, the humectant comprises glycerine, propylene glycol, dipropylene glycol, polypropylene glycol, polyethylene glycol, sorbitol, hydroxypropyl sorbitol, hexylene glycol, 1,3-butylene glycol, 1,2,6-hexanetriol, ethoxylated glycerin, propoxylated glycerin or mixtures thereof. Still more preferably, the humectant comprises glycerin. The cosmetic composition according to the invention may comprise from 0.1 to 30%, preferably from 5% to 25% and more preferably from 7% to 15% humectant by weight of the cosmetic composition.

Cosmetic compositions according to the invention may further comprise a hydrophilic vitamin component. The hydrophilic vitamin component may comprise any vitamin that fulfils the definition of "hydrophilic" provided herein. Preferably, the hydrophilic vitamin component comprises vitamin B₃, salts or esters thereof; pro-vitamin B₅, salts or esters thereof; vitamin C, salts or esters thereof; hydrophilic derivatives of vitamin E, such as tetramethylchromanol glucosides and 6-hydroxy-2,5,7,8-tetramethylehroman-2-caroxylic acid (TROLOX^{™}); or mixtures thereof. Preferably, the hydrophilic vitamin component comprises vitamin B₃, salts or esters thereof; vitamin B₅, salts or esters thereof; or mixtures thereof. More preferably, the hydrophilic vitamin component comprises vitamin B₃, salts or esters thereof. The cosmetic composition according to the invention may comprise from 0.01 % to 10%, preferably from 0.1% to 9%, more preferably from 0.5% to 5% of the hydrophilic vitamin component by weight of the composition.

Cosmetic compositions of the present invention may further comprise a hydrophobic vitamin component. The hydrophobic vitamin component may comprise any vitamin that fulfils the definition of "hydrophobic" provided herein. Preferably, hydrophobic vitamin component comprises vitamin E and/or its hydrophobic derivatives, preferably tocopheryl nicotamide, vitamin D and/or its derivatives) or mixture thereof The cosmetic composition may comprise from 0.01% to 10%, preferably from 0.1% to 9%, more preferably from 0.5% to 5% of the hydrophobic vitamin component by weight of the composition.

Cosmetic compositions of the present invention may further comprise a hydrophobic botanical extract. The hydrophobic botanical extract may comprise any botanical extract that fulfils the definition of "hydrophobic" provided herein. Preferably, hydrophobic botanical extract comprises argan oil (*Argania Spinosa*), bilberry seed oil (*Vaccinium myrtillus*), blackcurrant seed oil (*Ribes Nigrum*), cotton seed oil (*Gossypium Herbaceum*), cranberry seed oil (*Vaccinium Macrocarpon*), cumin extract (comprising tetrahydrocurcumin), evening primrose oil (*Oenothera Biennis*), grape seed oil (*Vitis Vinifera*), grapefruit seed oil (*Citrus Grandis*), kiwi seed oil (*Actinidia Chinensis*), lavender (*Lavendular Augustafolia*), ligonberry seed oil (*Vaccinium Vitis-Idaea*), lime seed oil (*Citrus auranifolia*), linseed oil (*Linum usitatissimum*), liquorice root (*glycyrrhiza glabrai*), olive oil (*Olea Europaea*)*,* olive wax (*Olea Europaea*), organic grape seed oil (*Vitis Vinifera*), oriental popy seed oil (*Papaver Orientale*), meadowfoam seed oil (*Limnanthes alba*), neem leaf and/or bark oil (*azadirachta indica*), palm oil (*Elaeis guineenis*), papaya seed oil (*Carica Papaya*), passion fruit seed oil (*Passiflora edulis*), pumpkin seed oil (*Cuerbita pepo*), raspberry seed oil (*Rubus idaeus*), rosehip seed oil (*Rosa Canina*), rosemary oil (*Rosmarinus officinalis*), seabuckthorn seed oil (*Hippophae Rhamnoides*), sesame seed oil (*Sesamum indicum*), shea butter (*butyrospermum parkii*), soy extract, sweet almond oil (*Prunus amygdalus dulcis*), watermelon seed oil (*Citrullus vulgaris*), or mixtures thereof. The cosmetic composition may comprise from 0.0001% to 1%, preferably from 0.001% to 0.4%, and more preferably from 0.002% to 0.15% of the hydrophobic botanical extract by weight of the composition.

Cosmetic compositions according to the present invention may further comprise at least one sunscreen active, comprising a hydrophobic organic sunscreen active, a hydrophilic organic sunscreen active, an inorganic sunscreen or mixtures thereof. Suitable examples of sunscreens may be found in the CTFA international Cosmetic Ingredient Dictionary and Handbook, 7th edition, volume 2 pp. 1672, edited by Wenninger and McEwen (The Cosmetic, Toiletry, and Fragrance Association, Inc., Washington, D. C., 1997).

Preferred hydrophobic organic suncreens suitable to be included in compositions according to the invention comprise alkyl β,β-diphenylacrylate and/or α-cyano β,β-diphenylacrylate derivatives, such as ethyl 2-cyano-3,3-diphenylacrylate, 2-ethylhexyl 2-cyano-3,3-diphenylacrylate; dibenzoylmethane derivatives, such as butyl methoxydibenzoylmethane, ethylhexyl methoxydibenzoylmethane, isopropyl dibenzoylmethane; benzophenone derivatives, such as benzophenone-1 (benzoresorcinol benxoresorcinol), benzophenone-2 (tetraydroxybenzophenone), benzophenone-3 (oxybenzone), benzophenone-4 (sulisobenzone), benzophenone-5 (sulisobenzone sodium), benzophenone-6 (dihydroxy dimethoxy benzophenone), benzophenone-7 (2-benzoyl-4-chlorophenol), benzophenone (8 dioxybenzone), benzophenone-9, benzophenone-10 (mexenone), benzophenone-11, benzophenone-12 (octabenzone); cinnamic derivatives, such as octyl methoxycinnamate (octinoxate), diethanolamine methoxycinnamate; salicylic derivatives, such as ethylhexyl salicylate (octisalate), triethanolamine salicylate, 3,3,5-trimethylcyclohexylsalicylate, homomenthyl salicylate (homosalate); camphor derivatives, such as 4-methylbenzylidene camphor (enzacamene); triazine derivatives, such hexylethyl triazone; anthranilate derivatives, such as menthyl anthranilate (meradimate); p-aminobenzoic acid derivatives, such as aminobenzoic acid (PABA), glyceryl FABA (lisadimate), octyl dimethyl PABA, ethyl dihydroxypropyl PABA; or mixture thereof. More preferably, hydrophobic organic suncreens comprise alkyl β,β-diphenylacrylate and/or α-cyano β,β-diphenylacrylate derivatives, such as ethyl 2-cyano-3,3-diphenylacrylate, 2-ethylhexyl 2-cyano-3,3-diphenylacrylate; dibenzoylmethane derivatives, such as butyl methoxydibenzoylmethane, ethylhexyl methoxydibenzoylmethane, isopropyl dibenzoylmethane. Still more preferably, hydrophobic organic sunscreens comprise 2-ethylhexyl 2-cyano-3,3-diphenylacrylate, butyl methoxydibenzoylmethane, or mixture thereof The cosmetic composition may comprise from 0.01% to 15%, preferably from 0.1% to 10%, and more preferably from 1 % to 8% of a hydrophobic organic sunscreen by weight of the composition.

Preferred hydrophilic organic suncreens suitable to be included in compositions according to the invention may comprise 2-phenylbenzimidazole-5-sulfonic acid (PBSA). The cosmetic composition according to the invention may comprise from 0.1% to 5%, preferably from 0.5% to 2.25%, and more preferably from 0.75% to 1.5% of a hydrophilic organic sunscreen by weight of the composition.

Cosmetic compositions according to the present invention may comprise at least one inorganic sunscreen active having a mean primary particle size less than to 200 nm, preferably less than 100 nm, and most preferably from 1 to 30 nm. Preferably, inorganic sunscreen actives comprise titanium dioxide, zinc oxide, or mixtures thereof. The cosmetic composition according to the invention may comprise from 1% to 15%, preferably from 1% to 10% of an inorganic sunscreen by weight of the composition.

Cosmetic compositions according to the present invention may further comprise at least one tanning active. Preferably, said tanning active is selected from dihydroxyacetone (DHA), salts, derivatives or tautomers thereof, or mixtures thereof. The cosmetic composition according to the invention may comprise from 0.01% to 10%, preferably from 0.05% to 5%, and more preferably from 0.1 % to 1% of at least one tanning active by weight of the composition.

Cosmetic compositions according to the present invention may further comprise at least one sugar amine. Said sugar amine can be synthetic or natural in origin and can be used as pure compounds or mixtures of compounds (e.g., extracts from natural sources or mixtures of synthetic materials). Preferably, sugar amine comprises glucosamine, N-acetyl glucosamine, mannosamine, N-acetyl mannosamine, galactosamine, N-acetyl galactosamine, isomers thereof (e.g., stereoisomers), salts thereof (e.g., HCl salt), or mixtures thereof. The cosmetic composition according to the invention may comprise from 0.01% to 15%, preferably from 0.1 % to 10%, and more preferably from 0.5% to 5% of at least one sugar amine by weight of the composition.

Cosmetic compositions according to the present invention may further comprise at least one hexaminidine compound, salts or derivatives thereof, or mixtures thereof. Preferably, hexamindine compound comprises compounds corresponding to the following chemical structure: 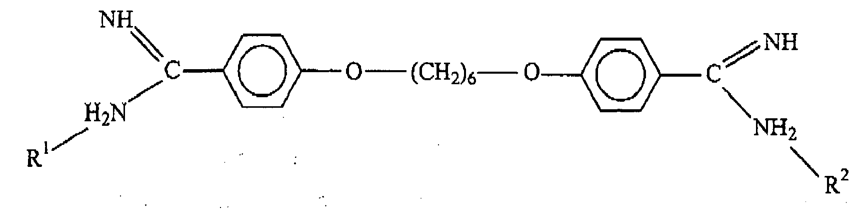 wherein R¹ and R² are organic acids (e.g., sulfonic acids, etc.). The cosmetic composition according to the invention may comprise from 0.001% to 10%, preferably from 0.01% to 5%, and more preferably from 0.02% to 2.5% of a hexaminidine compound by weight of the composition.

Cosmetic compositions, in the form of an oil-in-water emulsions, may further comprise at least one hydrophilic surfactant. Generally, hydrophilic surfactants help disperse and suspend the discontinuous phase within the continuous phase. Preferably, hydrophilic surfactants are selected from nonionic surfactants such as those known in the art. Other suitable surfactants useful herein comprise a wide variety of cationic, anionic, zwitterionic, and amphoteric surfactants such as are known in the art. See, e.g., McCutcheon's, Detergents and Emulsifiers, North American Edition (1986), published by Allured Publishing Corporation. The hydrophilic surfactants useful herein can contain a single surfactant, or any combination of suitable surfactants. The exact surfactant (or surfactants) chosen will depend upon the pH of the composition and the other components present. The cosmetic composition according to the invention may comprise from 0.05% to 10%, preferably from 1% to 6%, and more preferably from 1% to 3% of at least one hydrophilic surfactant by weight of the composition.

The oil phase may comprise at least one oily component such as a natural or synthetic oils selected from mineral, vegetable, and animal oils, fats and waxes, fatty acid esters, fatty alcohols, fatty acids and mixtures thereof. For example, the oil phase may comprise saturated and unsaturated fatty alcohols such as behenyl alcohol, cetyl alcohol and stearyl alcohol and hydrocarbons such as mineral oils or petrolatum.

Cosmetic compositions according to the present invention may further comprise at least one emollient material including branched chain hydrocarbons having a weight average molecular weight of from 100 to 15,000, preferably from 100 to 1000. Preferably branched chain hydrocarbons comprise isododecane, isohexadecane, isoeicosane, isooctahexacontane, isohexapentacontahectane, isopentacontaoctactane, petrolanum, isopropyl palmitate, isopropyl isostearate or mixture thereof. The composition according to the invention may comprise from 0.1% to 15%, preferably from 1% to 7%, more preferably from 3% to 5% of at least one emollient material by weight of the composition.

Cosmetic compositions according to the present invention may further comprise at least one particulate material. Particulate materials may comprise particulate materials as such, colored and uncolored pigments, interference pigments, inorganic powders, composition powders, optical brightener particles, or mixture thereof, said materials being known in the art. These particulate materials do not comprise inorganic sunscreen active. Preferably, particulate materials as such comprise tree-flowing, solid (i.e. not hollow) materials that are insoluble in both water and oil, with a median particle size of from 0,1µm to 75 µm, more preferably from 0,2µm to 30µm, and with a refractive index of from 1.3 to 1.7, said materials being dispersed in the composition. Suitable particulate materials are organic or organosilicone or inorganic. The composition according to the invention may comprise from 0.01% to 20%, preferably from 0.05% to 10%, and more preferably from 0.1% to 5% particulate materials by weight of the composition.

The oil-in-water emulsion according to the present invention may further comprise a structuring agent to assist in the formation of a liquid crystalline gel network structure.

Preferably, structuring agents are selected from stearic acid, palmitic acid, stearyl alcohol, cetyl alcohol, behenyl alcohol, steareth-2, steareth-21, or mixtures thereof. The cosmetic composition according to the invention may comprise from 0.5% to 20%, preferably from 1% to 10%, more preferably from 1% to 5% of a structuring agent by weight of the composition.

A variety of additional optional ingredients may be incorporated into the compositions of the present invention. Non-limiting examples of these additional ingredients include additional skin care actives such as bisabolol, dialkanoyl hydroxyproline compounds, farnesol, flavonoids, guanidine (e.g., amino guanidine), N-acyl amino acid compounds, peptides (e.g. Matrixyl [Pal-KTTKS]), phytantriol, phytosterols, salicylic compounds, urea as well as compounds such as anti-acne compounds (e.g. resorcinol, erythromycin); antioxidants compounds (e.g., phytosterols, lipoic acid); skin soothing and healing agents; anti-wrinkle/anti-atrophy actives; conditioning agents; anti-inflammatory agents; skin lightening agents; antimicrobial/antibacterial/antifungal actives; chelators and sequestrants; and agents suitable for aesthetic purposes such as essential oils, fragrances, skin sensates, opacifiers, aromatic compounds (e.g., clove oil, menthol, camphor, eucalyptus oil, and eugenol), preservatives, or mixtures thereof.

According to a further aspect of the invention, a process is provided for preparing a cosmetic composition in the form of an oil-in-water emulsion comprising a continuous aqueous phase comprising (1) a cross-linked co-polymer and (2) at least one hydrophilic botanical extract, wherein that process comprises the step of adding the hydrophilic botanical extract to the composition, the temperature of the composition at the time of addition being less than 50°C, preferably less than 40°C.

Thickeners are added to emulsions in order to stabilize these emulsions. That addition is generally performed at high temperature, preferably more than 50°C, in order to obtain a homogeneous emulsion. In fact, the higher the temperature is, the lower the viscosity. Once thickeners have been added, it may be necessary to add remaining components (actives ingredients, botanical extracts, etc.) while the emulsion has a viscosity less than 20,000cps and so, while the temperature is still high. As the viscosity increases (particularly when the viscosity is more than 50,000cps), it is more difficult to dissolve and/or to disperse homogenously the remaining components. The addition of remaining ingredients at high temperature may present drawbacks however, such as a partial or total denaturation of active ingredients and/or botanicals. Unexpectedly, the inventors have found that the cross-linked co-polymer according to the invention may be added at low temperature, preferably less than 50°C, more preferable less than 40°C, without immediately increasing the viscosity of the composition, i.e. the viscosity is about 20,000cps and increases slowly up to 50,000cps to 80,000cps. Consequently, remaining components, preferably botanical extracts, may be further added at the composition at low temperature.

The composition of the present invention may be useful for treating a number of mammalian keratinous tissue conditions. Such cosmetic treatment of keratinous conditions can include prophylactic and therapeutic regulation. More specifically, such cosmetic composition can be useful for regulating the skin barrier function, the skin tone/eveness, the skin texture, for providing antioxidant effects, etc.
For instance, the cosmetic composition of the present invention can be for regulating visible and/or tactile discontinuities in mammalian keratinous tissue, including discontinuities in skin texture and color.
Regulating keratinous tissue conditions can involve topically applying to the keratinous tissue a safe and effective amount of the composition of the present invention. The amount of the composition that is applied, the frequency of application, and the period of use will vary widely depending upon the level of components of the given composition and the level of regulation desired, e.g., in view of the level of keratinous tissue damage present or expected to occur.

### Examples

The compositions detailed below comprise four different phases of components (cf. table below). Phase A and phase B are heated separately at 75°C under agitation at 150 rpm. Phase B is then added to A, and the obtained mixture is mixed at 13,000 rpm (using a high shear mixer such as ultra tarax® to emulsify). Said mixture is then cooled to 50°C and phase C is added. The obtained mixture is cooled to 40°C and phases D (comprising the co-polymer according to the invention) and then E (comprising botanicals) are added.

| **Ingredient** | **Ph** | **1** | **2** | **3** | **4** | **5** | **6** | **7** | **8** |
|---|---|---|---|---|---|---|---|---|---|
| Deionised water | B | Qs | Qs | Qs | Qs | Qs | Qs | Qs | Qs |
| Glycerin | B | 10.0 | 10.0 | 10.0 | 10.0 | 10.0 | 7.0 | 10.0 | 10.0 |
| Octyl methoxycinnamate | A | | | | | | | | 2.00 |
| Octyl Salicylate (Ethylhexyl Salicylate) | A | 4.00 | | | | | | | |
| Avobenzone (Butyl methoxydibenzoylmethane) | A | 2.00 | | | | | | . | |
| Octocrylene | A | 1.00 | | | | | | | |
| Phenylbenzimidazole Sulfonic Acid | B | 1.00 | | | | | | | |
| Capric/Caprylic Triglycerides and Sodium Acrylates Copolymer | D | 3.00 | 2.50 | 2.00 | 1.80 | 2.25 | 1.50 | 2.25 | 2.00 |
| Xanthan gum | B | | | | | | 0.06 | | |
| Niacinamide | B | 5.00 | 3.50 | 2.00 | 2.00 | 2.00 | 1.00 | 2.00 | 5.00 |
| D-Panthenol | B | 1.25 | 1.00 | 0.75 | 0.50 | 0.50 | 0.25 | 0.50 | 1.25 |
| Tocopheryl Acetate | A | 0.50 | 0.05 | 0.25 | 0.25 | 0.50 | 0.25 | 0.25 | 0.50 |
| Vitamin C | E | 0.00 | 3.00 | 1.00 | 0.00 | 0.00 | 0.00 | 0.00 | 0.00 |
| Magnesium Ascorbyl Phosphate | E | 0.50 | | | 0.50 | 0.25 | 0.00 | 0.00 | 0.50 |
| Sodium Ascorbyl Phosphate | E | | | | | | 0.50 | 0.05 | |
| Dimethicone and Dimethiconol | C | 2.00 | 1.00 | | 2.00 | 2.00 | 2.00 | 2.00 | 1.50 |
| Dimethicone 350 CS | C | | 1.00 | 0.50 | | | | | 0.50 |
| Cyclopentasiloxane & Dimethicone copyol | C | | | 2.00 | | | | | |
| Petrolatum | A | | | | | 0.10 | | | 1.50 |
| Isopropyl Isostearate | A | | 1.50 | 1.33 | 1.33 | | | 1.33 | |
| Isopropyl palmitate | A | 1.00 | | | | 1.40 | 1.00 | | 1.50 |
| Cetyl Alcohol | A | 1.00 | 0.80 | 0.90 | 0.80 | 0.70 | 1.00 | 0.80 | 2.00 |
| Behenyl Alcohol | A | | | | 0.60 | 0.50 | | | 0.40 |
| Stearyl Alcohol 95% | A | 0.75 | 0.45 | 0.60 | 0.40 | 0.90 | 0.50 | 0.55 | 1.00 |
| Stearic Acid | A | 0.10 | 0.10 | 0.10 | 0.10 | 0.10 | 0.10 | 0.10 | 0.30 |
| Sorbitan Stearate | A | 0.60 | 0.70 | 1.00 | 0.90 | 1.30 | 0.50 | 0.90 | |
| Cetearyl Glucoside & Cetearyl Alcohol | A | 0.40 | 0.10 | 0.20 | 0.30 | 0.05 | 0.50 | 0.10 | |
| Polyethylene | C | 1.00 | 1.00 | 2.00 | 1.50 | 0.20 | | 0.80 | 1.00 |
| Aluminum Starch Octenylsuccinate | C | 0.50 | 1.00 | 2.00 | 0.50 | 0.20 | 1.00 | | |
| CI 77891 titanium dioxide | C | 0.10 | 0.50 | 0.25 | 0.30 | | | | |
| Perfume | E | | | | | | 0.20 | | |
| Disodium EDTA | B | 0.10 | 0.10 | 0.10 | 0.10 | 0.10 | 0.10 | 0.10 | 0.10 |
| PEG-100 Stearate | A | 0.10 | 0.10 | 0.10 | 0.10 | 0.10 | 0.10 | 0.10 | 0.30 |
| Ethylparaben | A | 0.10 | 0.20 | 0.20 | 0.20 | 0.20 | 0.20 | 0.20 | 0.10 |
| Methylparaben | B | 0.20 | | | | | | | 0.20 |
| propyl Paraben | A | 0.10 | 0.10 | 0.10 | 0.10 | 0.10 | 0.10 | 0.10 | 0.10 |
| Benzyl Alcohol | E | 0.25 | 0.25 | 0.25 | 0.25 | 0.25 | 0.25 | 0.25 | 0.25 |
| Sodium Hydroxide | B | | 0.04 | 0.03 | 0.02 | 0.04 | 0.02 | 0.01 | 0.07 |
| Triethanolamine | B | 0.89 | | | | | | | 0.89 |
| Laureth-7 | A | 0.02 | 0.02 | 0.02 | 0.02 | 0.02 | 0.02 | 0.02 | 0.02 |
| Oleth-3 | A | 0.07 | 0.05 | 0.05 | 0.05 | 0.05 | 0.05 | 0.05 | 0.07 |
| Fruitapone | E | 5.00 | 1.00 | 0.10 | 0.01 | 0.01 | 0.25 | 0.05 | 0.05 |
| Rose Hip Extract | E | 0.10 | 0.10 | 0.10 | 0.10 | | | 0.10 | 0.10 |
| Grape Seed extract | E | | | | | | 0.05 | | |
| Ginseng | E | | | | | | 0.10 | | |
| Lemon Grass | E | | | | | | 0.05 | | |
| Wheat Germ Extract | E | | | | | 0.05 | | | |
| Calendula Extract | E | 0.05 | 0.05 | 0.05 | 0.05 | | | 0.05 | 0.05 |
| Honey | E | | | | | 0.05 | | | |
| Sweet Almond Oil | E | 0.05 | 0.05 | 0.05 | 0.05 | | | 0.05 | 0.05 |
| | | **100** | **100** | **100** | **100** | **100** | **100** | **100** | **100** |

## Claims

1. A cosmetic composition in the form of an oil-in-water emulsion comprising a continuous aqueous phase comprising (1) a cross-linked co-polymer comprising; i) from 35% to 94.5% water-soluble anionic acrylic monomers by weight of the cross-linked co-polymer; ii) from 5% to 65% water-soluble non-ionic acrylate monomers by weight of the cross-linked co-polymer; and iii) from 0.005% to 0.5% of a bifunctional monomeric cross-linking agent by weight of the cross linked co-polymer, and (2) a hydrophilic botanical extract.

2. A composition according to claim 1, wherein said cross-linked co-polymer comprises (i) water-soluble anionic acrylic-based monomers selected from acrylic acid, methacrylic acid or mixtures thereof, (ii) water-soluble non-ionic acrylate monomers selected from acrylamide, methacrylamide, N-vinyl pyrolidone, water-soluble hydroxy-substituted acrylic or methacrylic esters or mixtures thereof, and (iii) bifunctional monomeric cross-linking agents selected from di-, tri- and tetraethylenically unsaturated materials such as methylene bis acrylamide, divinylpyrroline and allyl(meth)acrylate or mixtures thereof.

3. A composition according to claim 1 or 2 comprising from 0.01% to 10%, preferably from 0-1% to 7%, more preferably from 1% to 3% of cross-linked co-polymer by weight of the composition.

4. A composition according to any of preceding claims, wherein said hydrophilic botanical extract comprises a hydrophilic extract of aloe vera leaf, argan tree leaf and/or fruit, baobab or cream of tartar tree leaf, bearberry leaf, brahmi whole plant, butcher's broom, centella asiatica, chamomile, chestnut, clary sage leaf, eyebright leaf, ginkgo biloba leaf, grape fruit, leaf and/or seed, green tea leaf, honey, horestail leaf, horse chestnut seed, Indian cress whole plant, lemongrass plant leaf, linden wood, leaf and/or bark, liquorice root, marigold flower, mimosa leaf and/or bark, mulberry leaf, neem leaf and/or bark, nettle leaf, oat germ and/or bran, oergano leaf, olive leaf and/or fruit, panax ginseng root extract, plantago leaf, propolis, rose hip fruit, rosemary leaf, sage leaf, st. Mary's thisle, seabuckthorn, sesame seed, sweet manadarin peel, wheat bran and/or germ, willow bark, witch hazel, or mixtures thereof, more preferably an hydrophilic extract of seabuckthom, rose hip fruit, ginko biloba leaf, panax ginseng root or mixtures thereof.

5. A composition according to any of preceding claims comprising from 0.0001% to 1%, preferably from 0.001% to 0.4%, and more preferably from 0.002% to 0.15% of hydrophilic botanical component by weight of the composition.

6. Composition according to any one of the preceding claims further comprising a humectant comprising polyhydric alcohol, preferably glycerine, propylene glycol, dipropylene glycol, polypropylene glycol, polyethylene glycol, sorbitol, hydroxypropyl sorbitol, hexylene glycol, 1,3-butylene glycol, 1,2,6-hexanetriol, ethoxylated glycerin, propoxylated glycerin or mixtures thereof.

7. Cosmetic composition according to claim 6 comprising a humectant comprising glycerin.

8. Composition according to any one of the preceding claims further comprising a hydrophilic vitamin component, preferably comprising vitamin B₃, salts or esters thereof; pro-vitamin B₅, salts or esters thereof; vitamin C, salts or esters thereof; hydrophilic derivatives of vitamin E; or mixtures thereof; more preferably vitamin B₃, salts or esters thereof; vitamin B₅, salts or esters thereof, or mixtures thereof.

9. A composition according to any of preceding claims further comprising a hydrophobic vitamin component, preferably selected from vitamin E and/or its hydrophobic derivatives, vitamin D and/or its derivatives, or mixtures thereof.

10. A composition according to any one of the preceding claims further comprising a hydrophobic botanical component, preferably comprising argan oil, bilberry seed oil, blackcurrant seed oil, cotton seed oil, cranberry seed oil, cumin extract, evening primrose oil, grape seed oil, grapefruit seed oil, kiwi seed oil, lavender, ligonberry seed oil, lime seed oil, linseed oil, liquorice root, olive oil), olive wax, organic grape seed oil, oriental popy seed oil, meadowfoam seed oil, neem leaf and/or bark oil, palm oil, papaya seed oil, passion fruit seed oil, pumpkin seed oil, raspberry seed oil, rosehip seed oil, rosemary oil, seabuckthorn seed oil, sesame seed oil, shea butter, soy extract, sweet almond oil, watermelon seed oil, or mixtures thereof.

11. Composition according to any one of the preceding claims further comprising a hydrophobic organic sunscreen, preferably alkyl β,β-diphenylacrylate and/or α-cyano β,β-diphenylacrylate derivatives, dibenzoylmethane derivatives, benzophenone derivatives, cinnamic derivatives, salicylic derivatives, camphor derivatives, triazine derivatives, anthranilate derivatives, p-aminobenzoic acid derivatives, or mixture thereof, more preferably alkyl β,β-diphenylacrylate and/or α-cyano β,β-diphenylacrylate derivatives, dibenzoylmethane derivatives, or mixture thereof, still more preferably 2-ethylhexyl 2-cyano-3,3-diphenylacrylate, butyl methoxydibenzoylmethane, or mixtures thereof.

12. Composition according to any one of the preceding claims further comprising a hydrophilic organic sunscreen, preferably comprising 2-phenylbenzimidazole-5-sulfonic acid.

13. Composition according to any one of the preceding claims further comprising a tanning active, preferably dihydroxyacetone, salts, derivatives or tautomers thereof, or mixtures thereof.

14. Composition according to any one of the preceding claims further comprising at least one sugar amine, preferably glucosamine, N-acetyl glucosamine, mannosamine, N-acetyl mannosamine, galactosamine, N-acetyl galactosamine, isomers thereof, salts thereof, or mixtures thereof.

15. Composition according to any one of the preceding claims further comprising at least one hexaminidine compound, salts or derivatives thereof, or mixtures thereof.

16. Process suitable for preparing a composition according to any one of the preceding claims **characterized in that** it comprises the step of adding the hydrophilic botanical extract to the composition, the temperature of the composition at the time of addition being less than 50°C, preferably less than 40°C.

17. Use of a cosmetic composition according to claims 1 to 15 for regulating the condition of the skin.
